Europäisches Patentamt

European Patent Office

Office européen des brevets

(11) Numéro de publication: **0 242 270**
**A1**

## (12) DEMANDE DE BREVET EUROPEEN

(21) Numéro de dépôt: 87400767.7

(22) Date de dépôt: 07.04.87

(51) Int. Cl.⁴: **C 12 N 5/00**
A 61 K 37/12, A 61 L 27/00,
A 61 L 15/04, C 12 N 7/00

(30) Priorité: 18.04.86 FR 8605590

(43) Date de publication de la demande:
21.10.87 Bulletin 87/43

(84) Etats contractants désignés:
AT BE CH DE ES GB GR IT LI NL SE

(71) Demandeur: INSTITUT MERIEUX Société anonyme dite:
17, rue Bourgelat
F-69002 LYON (FR)

(72) Inventeur: Tayot, Jean-Louis
1 rue des Greffières
F-69890 La Tour de Salvagny (FR)

Nguyen, Cuc
6 Chemin du Grand-Chêne
F-69260 Charbonnières Les Bains (FR)

(74) Mandataire: Lemoine, Michel et al
Cabinet Michel Lemoine et Bernasconi 13 Boulevard des
Batignolies
F-75008 Paris (FR)

(54) Procédé de fabrication de nappes de collagène, nappes obtenues et leurs applications.

(57) On réalise une couche confluente de cellules destinées à coloniser le collagène et l'on met en contact cette couche confluente de cellules avec une nappe de collagène en présence d'un milieu convenable, après quoi on laisse les cellules coloniser une partie au moins de ladite nappe et provoquer une rétraction de la nappe uniquement en épaisseur. Application à lapréparation de derme équivalent et à la culture de virus.

EP 0 242 270 A1

## Description

Procédé de fabrication de nappes de collagène, nappes obtenues et leurs applications.

La présente invention a trait à un procédé de fabrication de nappes de collagène du type de celles obtenues à partir d'une couche ou réseau de collagène habitée ou habitable par des cellules et pouvant notamment servir de peau artificielle, en particulier pour le traitement des brûlures. L'invention a également trait aux nappes obtenues.

Devant le problème posé par la reconstitution de la barrière cutanée des grands brûlés, on a cherché, en dehors de la mise en place d'autogreffes, disponibles en quantités insuffisantes chez les grands brûlés, ou d'allogreffes, se traduisant généralement par des phénomènes de rejet, à recouvrir la peau de nappes ou films synthétiques ou reconstitués à partir de collagène.

En conséquence, depuis plusieurs années, les recherches se sont orientées vers la mise au point d'une peau artificielle produite in vitro.

Le collagène, qui constitue le composant majeur de la matrice extra-cellulaire in vivo, a été largement étudié en gel tri-dimensionnel pour les cultures de cellules. Cela a été en particulier le cas pour les cultures de cellules hématopoïétiques (voir LANOTTE M. Terminal differentiation of hemopoietic cell clones cultured in tridimensional collagen matrix : in situ cell morphology and enzyme histochemistry analysis, Biol. Cell, 50, 107-120, 1984) ; pour des cellules folliculaires thyroïdiennes (voir CHAMBARD M., VERRIER B, GABRION J. and MAUCHAMP J. Polarity reversal of inside-out thyroid follicles cultured within collagen gel : reexpression of specific functions, Biol. Cell, 51, 315-326, 1984) ; et surtout pour les fibroblastes cutanés d'après le modèle de BELL. pour la production d'un derme équivalent (voir COULOMB B., DUBERTRET L., BELL E., MERRILL C., FOSSE M., BRETON-GORIUS J., PROST C. and TOURAINE R. Endogenous peroxidases in normal human dermis : a marker of fibroblast differentiation, J. Invest. Dermatol., 81 (1), 75-78, 1983, et COULOMB B., DUBERTRET L., BELL E. and TOURAINE R. The contractility of fibroblasts in a collagen lattice is reduced by corticosteroids, J. Invest. Dermatol., 82 (4), 341-344, 1984).

D'après ces derniers auteurs, seul un collagène de type I non pepsiné peut donner des propriétés rétractiles en présence de fibroblastes pour former un réseau manipulable de bonne solidité mécanique.

Un tel derme équivalent et son procédé de fabrication sont décrits dans le brevet US-A-4.485.096 et dans la demande de brevet WO 80/01350. Ces documents décrivent un derme artificiel humain obtenu en mélangeant du collagène provenant de la peau ou des tendons de veau ou de rat, c'est-à-dire de collagène animal de type I, avec des cellules de fibroblastes humains en présence d'un milieu de culture convenable. Le procédé consiste à former un mélange conduisant à un gel de collagène habité par des fibroblastes, ledit gel se contractant sous l'action des fibroblastes qui s'attachent aux fibres et provoquent l'agencement d'une structure en réseau. Le derme artificiel ainsi obtenu peut ensuite être ensemencé en surface avec des cellules épithéliales ou kératinocytes prélevés sur le brûlé lui-même.

Le développement de la colonisation des fibroblastes dans la structure constituée par le mélange gélifié de collagène et de cellules conduit à une contraction dans un rapport de 4 à 5 des dimensions et notamment du diamètre de la nappe tissulaire que l'on cherche à former, ce qui constitue un grave inconvénient car il est difficile et parfois impossible d'obtenir des nappes de collagène ayant les dimensions souhaitées. De plus, cette contraction se poursuit sur une longue durée, ce qui aboutit à des délais d'attente insupportables, si l'on veut utiliser des cellules provenant du brûlé lui-même.

La présente invention se propose de remédier à ces inconvénients et de fournir un procédé permettant l'obtention, dans des délais rapides, de nappes à base de collagène habitées ou habitables par des cellules.

Un autre objectif de l'invention est de fournir un procédé qui supprime pratiquement ou totalement la rétraction en diamètre de la nappe, permettant une utilisation plus rapide de la nappe en tant que peau artificielle et autorisant, en outre, la préparation de nappes de très grande surface, et ceci d'un seul tenant, ces nappes pouvant, en outre, avoir toute forme de contour souhaitée.

Un autre objectif de l'invention est de fournir un procédé permettant de réaliser des nappes présentant une densité particulièrement élevée de cellules habitant la nappe de sorte que la nappe peut être utilisée dans de nombreuses applications n'ayant rien à voir avec les dermes équivalents, tels que par exemple la culture de virus.

Un autre objectif encore de l'invention est de fournir un procédé qui permettre d'utiliser du collagène placentaire et notamment du collagène placentaire pepsiné d'origine humaine.

Un autre objectif encore de l'invention est de fournir des nappes de collagène habitables ou habitées par des cellules présentant tous les avantages précités.

L'invention a pour objet un procédé de préparation d'une nappe à base de collagène, habitée et/ou habitable par des cellules, dans lequel on laisse se développer des cellules dans un réseau de collagène, caractérisé en ce que l'on réalise une couche confluente desdites cellules et que l'on met en contact cette couche confluente de cellules avec une nappe de collagène, en présence d'un milieu convenable, après quoi on laisse les cellules coloniser une partie au moins de ladite nappe et provoquer une rétraction de la nappe uniquement en épaisseur.

Dans un mode de mise en oeuvre préféré, on réalise sur un support plat, une culture de cellules pour obtenir une couche confluente, de préférence une mono-couche, et on ajoute ensuite une solution de collagène venant se gélifier sur la couche confluente cellulaire.

Dans une autre forme de réalisation, on peut préparer une nappe de gel de collagène et venir la mettre en contact à plat avec une couche confluente de cellules.

De préférence, au moment où la couche cellulaire confluente et la nappe de collagène se trouvent mises en contact, la nappe de gel de collagène n'est pas habitée par des cellules mais, dans certains cas, elle peut être habitée et, notamment, par des cellules identiques ou différentes, par exemple dont le développement dans le collagène est plus lent que celui des cellules de la couche confluente.

On peut également disposer sur la nappe de collagène en cours de colonisation par la couche confluente de cellules, une ou plusieurs autres nappes superposées, notamment de collagène, ces dernières pouvant ou non être déjà habitées par des cellules.

On peut également ensemencer une surface de la nappe en cours de colonisation, ou à la fin de la colonisation, par des cellules épithéliales telles que notamment des kératinocytes.

Le collagène peut être un collagène animal de type I mais d'autres collagènes peuvent être utilisés dans l'invention, et notamment le collagène placentaire animal ou humain de type I + III, y compris le collagène placentaire pepsiné.

La durée de la colonisation du gel de collagène par les cellules de la couche confluente peut être variable et peut dépendre notamment de la densité en cellules recherchée, ainsi que de l'épaisseur initiale de la nappe de gel de collagène.

Cette colonisation se développe de préférence jusqu'à la fin, c'est-à-dire jusqu'à ce que l'épaisseur de la nappe colonisée soit réduite à une valeur constante, mais on peut également l'interrompre à tout moment si on le désire.

En général, le rapport des épaisseurs initiale et finale de la couche ou nappe de collagène colonisée à partir de la couche de cellules confluentes est compris entre 2 et 10.

Les cellules utilisables doivent pouvoir former une couche confluente et de préférence une mono-couche. Toutes les cellules susceptibles d'être cultiviées en couche confluente et de coloniser un réseau de collagène peuvent être utilisées. Ainsi, on peut utiliser, par exemple, outre les fibroblastes cutanés, d'autres fibroblastes, par exemple des fibroblastes provenant de poumon embryonnaire humain ou bien encore des chondrocytes du cartilage.

On peut notamment utiliser des lignées cellulaires et en particulier des lignées cellulaires humaines telles que, par exemple, les souches diploïdes MRC5 ou WI38.

De préférence, la nappe initiale de gel de collagène à coloniser présente une épaisseur comprise entre 2 et 6 mm. La concentration en collagène dans le gel est de préférence comprise entre 0,5 et 5 mg/ml. Le milieu de culture doit, bien entendu, être approprié aux cellules en couche confluente qui sont utilisées. Parmi les milieux de culture utilisables et qui sont classiques, on peut citer notamment le milieu de base DMEM (DULBECCO Modified EAGLE Medium) additionné de sérum de veau foetal ou nouveau-né.

Pour préparer un gel de collagène, on peut notamment mélanger, au milieu de culture concentré, enrichi de sérum de veau, une solution de collagène en eau stérile. On peut également préparer un gel complètement dépourvu de protéines animales, avec notamment l'albumine humaine et/ou un sérum humain contenant des facteurs de crossance.

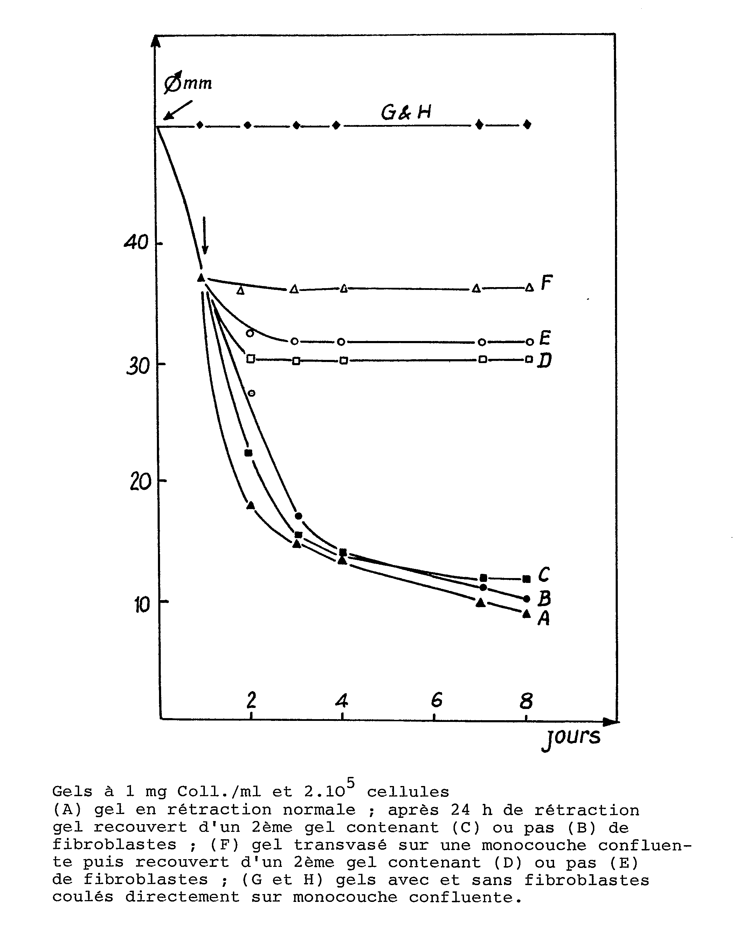

D'autres avantages et caractéristiques de l'invention apparaîtront à la lecture de la description suivante, faite à titre d'exemple non limitatif et se référant au dessin annexé dans lequel la figure unique représente des courbes graphiques comparatives de plusieurs variantes de l'invention et de la technique antérieure.

Exemple 1

300 kg de placenta humain sont broyés sous forme congelée pour donner des morceaux de quelques cm3. Le broyat est ensuite mélangé à 300 l d'une solution aqueuse contenant, au stade final, 8 % d'éthanol, 6 g/l de NaCl et 10 kg de cellulose. Après agitation à 10°C, l'ensemble est soumis à une opération de pressage avec un pressoir pour séparer le sang du tissu placentaire. On obtient ainsi 102 kg de tissu placentaire contenant 65 % d'eau.

Le tissu extrait du pressoir est agité dans 500 l de citrate de sodium 0,05 M à pH 7,2 pendant 30 mn à 10°C puis soumis au pressage pour récupérer le tissu. Un second lavage est effectué avec 500 l de cette même solution additionnée de 30 g/l de NaCl. Après récupération du tissu par pressage, un troisième lavage est effectué avec 500 l de citrate de sodium 0,05 M à pH 7,2. De préférence, le tissu ainsi lavé à pH neutre est ensuite soumis à plusieurs séquences de lavage à pH acide, toujours à 10°C.

Ces lavages peuvent être effectués dans des solutions d'acide citrique à pH acide 2,8 par addition d'HCl, puis d'acide formique 0,5 M pendant environ 15 heures et puis d'acide citrique additionné de NaCl à 20 g/l.

Le tissu placentaire ainsi lavé à pH acide et récupéré après pressage, a un aspect blanc qui témoigne d'une bonne élimination des pigments de sang placentaire initial. Le poids obtenu est de 82 kg.

Le tissu est soumis à une digestion enzymatique par la pepsine dans 500 l d'acide citrique 0,05 M à pH 2,8 contenant 300 g de pepsine pendant 15 heures à +10°C.

La suspension est ensuite diluée par addition de 500 l d'eau à +10°C et le pH ajusté à 7,5. Après 15 heures d'attente à +10°C le résidu tissulaire qui contient encore l'essentiel des collagènes I et III est séparé sur centrifugeuse. On obtient 103 kg de résidus.

Ce résidu est repris dans 800 l d'acide citrique 0,05 M à pH 2,8 contenant 300 g de pepsine, pendant 72 heures à +10°C. La suspension est alors diluée par addition de 200 l de NaCl à 50 g/l avant élimination du résidu tissulaire sur centrifugeuse.

Le pH du surnageant est ajusté à 7,5 par addition de soude pour dénaturer la pepsine. Après 2 heures d'attente, le pH est réajusté à 2,7 par addition de HCL 4N et laissé pendant 15 heures à +4°C, après quoi, on centrifuge la suspension. Le précipité est éliminé et le surnageant est additionné de NaCl 30 g/l. Après 15 heures à +4°C, le précipité de collagène formé est récupéré par centrifugation. On obtient 12 kg de précipité qui sont redissouts dans 600 l d'HCl 0,01 M et ensuite dilués avec 600 l de NaCl 24 g/l. Après 15 heures à +4°C, le précipité est éliminé par centrifugation et le surnageant est additionné de NaCl jusqu'à une concentration finale de 30 g/l.

On laisse se reposer pendant 15 heures à +4°C puis le précipité de collagène est recueilli par centrifugation. On récupère 8 kg de précipité.

Le précipité fait ensuite l'objet de plusieurs lavages à l'acétone puis est séché sous courant d'air stérile permettant d'obtenir 1 kg de fibres sèches et stériles de collagène contenant un mélange des types I et III.

Exemple 2

Les cellules utilisées sont les cellules diploïdes humaines MRC-5 entretenues sur le milieu de base DMEM à 10 % de sérum de veau foetal (SVF) ou nouveau-né. On réalise une culture de ces cellules sur boîte Falcon d'un diamètre de 5 cm jusqu'à obtenir une monocouche confluente.

On prépare, par ailleurs, une solution de collagène en poudre d'origine placentaire de type III + I en eau stérile.

On mélange la solution de collagène au milieu de culture concentré pour obtenir une suspension homogénéisée dont la teneur est la suivant :

| | |
|---|---|
| DMEM 5 x c | 1,00 ml |
| H2O | 0,55 ml |
| SVF | 0,45 ml |
| Collagène 2 mg/ml | 2,50 ml |

On coule très doucement cette suspension dans la boîte dont le fond est tapissé par la couche confluente de cellules. On complète avec 0,5 ml de DMEM enrichi de 10 % SVF pour obtenir une hauteur liquide de 3 mm.

Le gel de collagène polymérise rapidement à pH neutre et à 37°C.

On maintient la culture ainsi réalisée à température de 37°C sous atmosphère enrichie en $CO_2$ et l'on change périodiquement le milieu de culture.

On constate que la couche de gel de collagène se rétracte progressivement en épaisseur mais ne subit aucune rétraction en diamètre. Au bout de 4 jours, on obtient un film de collagène habité par les fibroblastes et dont l'épaisseur est de 1 mm. Le diamètre de ce film reste stable, comme le montre la courbe H sur le graphique. Ce film constitue une nappe qui présente des qualités mécaniques suffisantes pour pouvoir être manipulée et utilisée comme derme équivalent.

Exemple 3

On reprend l'exemple 2 avec cette différence qu'avant de couler, dans la boîte déjà tapissée de la couche confluente de cellules, la suspension destinée à fournir la couche de gel de collagène, on ajoute à la suspension 0,5 ml de milieu DMEM contenant 2 x $10^5$ cellules MRC-5 trypsinées en milieu DMEM à 10 % de sérum de veau foetal.

Comme on le voit sur la courbe G du graphique, la culture se poursuit sans aucune rétraction en diamètre. Seule l'épaisseur de la nappe diminue comme dans l'exemple 2.

Exemple 4

On réalise un gel de collagène ayant la composition prévue à l'exemple 2 que l'on laisse polymériser de façon à obtenir une couche ou nappe de gel ayant une épaisseur de 3 mm.

On prépare séparément, dans une boîte Falcon, une couche confluente de cellules MRC-5 entretenues en milieu DMEM à 10 % de sérum de veau foetal. Une fois la couche confluente obtenue, on transvase délicatement la couche de collagène gélifiée de façon à l'amener en superposition sur la couche confluente de cellules.

On constate que les cellules se mettent à coloniser le collagène et il se produit une rétraction en épaisseur de la nappe de gel de collagène, l'épaisseur se stabilisant au bout de 7 jours à environ 0,5 mm. Aucune rétraction en diamètre n'a lieu.

4

Exemple 5

Dans une boîte Falcon d'un diamètre de 5 cm, on met en place une suspension ayant la composition décrite à l'exemple 2 et on reprend des cellules MRC-5 trypsinées en milieu DMEM à 10 % de sérum de veau foetal. On ajoute 0,5 ml de milieu contenant les cellules à la suspension destinée à former le gel. On homogénéise la solution obtenue et on incube à 37°C sous $CO_2$. On évalue périodiquement la contraction en diamètre du gel. On constate que, comme on le voit sur la courbe A, le diamètre de 5 cm se trouve réduit à environ 1 cm au bout de huit jours.

Si, sur une autre boîte préparée de façon identique, on recouvre le gel tel qu'il vient d'être défini à la fin du premier jour, par une deuxième couche de gel, contenant également des fibroblastes MRC-5, on obtient une courbe de rétraction en diamètre (courbe C) pratiquement analogue à la courbe A. De même, si sur une troisième boîte on superpose à la fin du premier jour une deuxième couche ne contenant cette fois pas de fibroblastes, on obtient également, comme le montre la courbe B, une rétraction progressive en diamètre comparable à l'allure de la courbe A.

Exemple 6

On prépare une boîte Falcon avec une suspension de collagène contenant des fibroblastes comme dans l'exemple 5.

A la fin du premier jour, on transvase le gel sur une mono-couche confluente ; on constate que la rétraction en diamètre s'arrête rapidement comme le montre la courbe F. Seule la rétraction en épaisseur se poursuit.

Exemple 7

On reprend l'exemple 6 et, immédiatement après avoir transvasé le gel sur la couche confluente, on recouvre le gel d'un deuxième gel contenant des fibroblastes ; on observe une courbe de rétraction représentée en D dont le diamètre se stabilise rapidement.

Exemple 8

Dans le cas où, à la différence de l'exemple 7 le second gel de recouvrement ne contient pas de fibroblastes, on observe aussi, comme le montre la courbe E, un arrêt rapide de la rétraction en diamètre.

Exemple 9

On reproduit les opérations décrites dans les exemples 2 à 8 avec un gel de collagène placentaire ne contenant que des protéines humaines en remplacement du sérum de veau foetal (SVF).
La composition du gel est la suivante :
DMEM 5 x c : 1 ml
$H_2O$ : 0,45 ml
Albumine humaine 50 mg/ml : 0,5 ml
Sérum humain :0,05 ml
Collagène I + III 2 mg/ml : 2,5 ml.

Les cellules trypsinées, reprises en DMEM + 10% SVF, sont centrifugées 1 mn à 1000 g à +4°C. Ensuite on reprend le culot cellulaire en milieu DMEM sans sérum de veau. On dépose 0,5 ml de la suspension cellulaire ($2 \times 10^5$ cellules) dans la suspension de collagène.

L'ensemble est coulé dans des boîtes de 5 cm de diamètre selon les conditions décrites aux exemples 2 à 8.
Les résultats sont identiques.

Exemple 10

On reprend l'exemple 9, avec un gel de collagène placentaire appauvri davantage en protéines humaines.
La composition du gel est la suivante :
DMEM 5 x c : 1 ml
$H_2O$ : 0,95 ml
Sérum humain : 0,05 ml
Collagène I + III (2 mg/ml) : 2,5 ml.
Les résultats sont identiques.

Exemple 11

On reprend l'exemple 2 avec une suspension de collagène bovin acido-soluble de type I (PANCOGENE, produit commercialisé par SADUC-Chaponost-France) à la place du collagène placentaire I + III pepsiné.
Les résultats sont identiques.

Exemple 12

On reprend l'exemple 2 en utilisant une monocouche confluente de fibroblastes cutanés développée à partir d'une biopsie de peau humaine, à la place des cellules MRC5.
Les résultats sont identiques.

Exemple 13

On reprend l'exemple 2 en utilisant une monocouche de chondrocytes de cartilage humain, à la place des cellules MRC5.

Les résultats sont identiques.

## Revendications

1. Procédé de préparation d'une nappe à base de collagène, habitée etou habitable par des cellules, dans lequel on laisse se développer des cellules dans un réseau de collagène, caractérisé en ce que l'on réalise une couche confluente desdites cellules et que l'on met en contact cette couche confluente de cellules avec une nappe épaisse de collagène, en présence d'un milieu convenable, après quoi on laisse les cellules coloniser une partie au moins de ladite nappe et provoquer une rétraction de la nappe uniquement en épaisseur.

2. Procédé selon la revendication 1, caractérisé en ce que l'on réalise, sur un support plat, une culture de cellules pour obtenir une couche confluente et que l'on ajoute ensuite une solution de collagène capable de se gélifier sur la couche confluente cellulaire.

3. Procédé selon la revendication 1, caractérisé en ce que l'on réalise, sur un support plat, une culture de cellules pour obtenir une couche confluente, et que l'on met ladite couche en contact à plat avec une nappe de gel de collagène.

4. Procédé selon l'une quelconque des revendications 1 à 3, caractérisé en ce que l'on met en contact la couche confluente de cellules avec une nappe de collagène déjà habitée par des cellules.

5. Procédé selon l'une quelconque des revendications 1 à 3, caractérisé ence que l'on met en contact la couche confluente de cellules avec une nappe de collagène non habitée par des cellules.

6. Procédé selon l'une quelconque des revendications 1 à 5, caractérisé en ce que l'on dispose sur la nappe de collagène en cours de colonisation par la couche confluente de cellules, une ou plusieurs autres nappes à base de collagène.

7. Procédé selon l'une quelconque des revendications 1 à 6, caractérisé en ce que l'on ensemence une surface d'une nappe de collagène en cours de colonisation, ou après la colonisation, par des cellules épithéliales.

8. Procédé selonl'une quelconque des revendications 1 à 7, caractérisé en ce que l'on utilise un collagène placentaire, notamment pepsiné.

9. Procédé selon l'une quelconque des revendications 1 à 8, caractérisé en ce que le rapport des épaisseurs initiale et finale de la couche ou nappe de collagène colonisée à partir de la couche de cellules confluente est compris entre 2 et 10.

10. Procédé selon l'une quelconque des revendications 1 à 9, caractérisé en ce que l'on utilise comme cellules confluentes, des fibroblastes diploïdes humains, notamment de type MRC5.

11. Procédé selon l'une quelconque des revendications 1 à 9, caractérisé en ce que l'on utilise comme cellules confluentes des chondrocytes de cartilage.

12. Procédé selon l'une quelconque des revendications 1 à 11, caractérisé en ce que l'on utilise un collagène d'origine placentaire de type III + I notamment pepsiné.

13. Procédé selon l'une quelconque des revendications 1 à 12, caractérisé en ce que l'on utilise comme milieu de culture, le milieu de base DMEM additionné de sérum de veau foetal ou nouveau-né.

14. Procédé selon l'une quelconque des revendications 1 à 12, caractérisé en ce que l'on utilise comme milieu de culture l'albumine humaine pour réaliser un gel ne contenant que des protéines humaines.

15. Nappe de collagène obtenue par le procédé selon l'une quelconque des revendications 1 à 14, caractérisée par le fait qu'elle a subi une rétraction uniquement en épaisseur.

16. Application de la nappe de collagène selon la revendication 15 à la réalisation de derme équivalent.

17. Application du procédé selon l'une quelconque des revendications 1 à 14 ou de la nappe selon la revendica tion 15, à la culture de virus.

Gels à 1 mg Coll./ml et $2.10^5$ cellules
(A) gel en rétraction normale ; après 24 h de rétraction
gel recouvert d'un 2ème gel contenant (C) ou pas (B) de
fibroblastes ; (F) gel transvasé sur une monocouche confluente puis recouvert d'un 2ème gel contenant (D) ou pas (E)
de fibroblastes ; (G et H) gels avec et sans fibroblastes
coulés directement sur monocouche confluente.

## DOCUMENTS CONSIDERES COMME PERTINENTS

| Catégorie | Citation du document avec indication. en cas de besoin. des parties pertinentes | Revendication concernée | CLASSEMENT DE LA DEMANDE (Int Cl. 4) |
|---|---|---|---|
| X | WO-A-8 304 177 (MASSACHUSSETS INSTITUTE OF TECHNOLOGY)<br><br>* Pages 1-3; pages 16,17; exemples 1,9-11 * | 1,7,9-11,15, 16 | C 12 N 5/00<br>A 61 K 37/12<br>A 61 L 27/00<br>A 61 L 15/04<br>C 12 N 7/00 |
| X,D | WO-A-8 001 350 (MASSACHUSETTS INSTITUTE OF TECHNOLOGY)<br>* Revendications * | 1,7,9-11,16 | |
| X | BIOLOGICAL ABSTRACTS, vol. 78, no.6, 1984, pages 4963, résumé no. 44027, Philadelphia, P.A., US; F. GRINNELL et al.: "Reorganization of hydrated collagen lattices by human skin fibroblasts", & J. CELL. SCI. 66(0): 51-64, 1984<br>* Résumé * | 1-3,9, 15 | |
| Y | Idem | 4-6,8, 12 | DOMAINES TECHNIQUES RECHERCHES (Int. Cl.4)<br><br>C 12 N<br>A 61 L<br>A 61 K |
| Y | EP-A-0 080 956 (FONDATION MERIEUX)<br>* Page 10; revendications 7-10 * | 8,12 | |
| Y | WO-A-8 203 764 (MASSACHUSSETTS INSTITUTE OF TECHNOLOGY)<br>* Page 3, lignes 9-26; page 4, lignes 1-4; revendications * | 4-6 | |

Le présent rapport de recherche a été établi pour toutes les revendications

| Lieu de la recherche | Date d'achèvement de la recherche | Examinateur |
|---|---|---|
| LA HAYE | 18-05-1987 | SKELLY J.M. |